# EUROPEAN PATENT SPECIFICATION

(11) **EP 1 202 712 B1**
(45) Date of publication and mention of the grant of the patent: **09.11.2005**
(21) Application number: 00957351.0
(22) Date of filing: 09.08.2000
(51) Int. Cl.: A61K 7/50, A61K 7/155, B65H 35/00

(54) **TAPE STRIPPING SYSTEM AND METHOD**
STREIFENSPENDER UND METHODE
SYSTEME DE BANDE D'ARRACHAGE ET PROCEDE S'Y RAPPORTANT

(30) Priority: 09.08.1999 US 147916 P
(43) Date of publication of application: 08.05.2002
(73) Proprietor: ZELICKSON, Brian D., Minneapolis, MN 55416 (US); KIST, David A., Minneapolis, MN 55414 (US)
(72) Inventor: ZELICKSON, Brian D., Minneapolis, MN 55416 (US); KIST, David A., Minneapolis, MN 55414 (US)
(74) Representative: Howe, Steven
(86) International application number: PCT/US2000/021795
(87) International publication number: WO 2001/010404

(56) References cited:
- DE-U- 29 803 828
- FR-A- 2 063 743
- US-A- 4 447 482
- US-A- 5 383 900
- US-A- 5 685 833
- US-A- 6 136 200

## Description

The invention relates to apparatus and methods for cleaning and rejuvenating skin.

Various environmental and natural occurrences result in blemishes and wrinkles to human skin. For example, sun exposure, chronic irritation due to many different causes, and neglect may all cause skin problems. Recent advances in laser and chemical treatments have been successful in improving the appearance of the treated skin but these methods are rather labour intensive and can be traumatic.

One approach by medical practitioners includes the use of micro-dermabrasion techniques to treat skin blemishes. Although quite effective in certain applications, this technique can be risky and requires specially licensed operators.

Another approach by medical and cosmetic practitioners includes the use of adhesive-type tape applied to the patient's skin so that when the tape is removed the tape also removes an upper portion of the skin. This form of procedure is referred to as barrier disruption. Some analyses have suggested that barrier disruption by cellophane tape stripping triggers a cascade of biochemical activity which serves to "turn over" structural proteins that constitute human skin. For example, such tape stripping may stimulate the production of keratins 6,16,17 and reduce the amount of keratins 5 and 14, while also prematurely expressing involucrin. Yet another analysis suggests that tape stripping increases the rate of transepidermal water loss by 100 times the normal rate. In yet another analysis, tape stripping is suggested as having a regulatory effect upon fatty acid transport proteins and fatty acyl CoA synthase.

This data suggests that biochemical activity occurs following tape stripping which may be related to long term skin restructuring. An immediate consequence is the removal of redundant scale, plaques, and comedones which results in a rejuvenated appearance.

US-A-5383900 discloses a tape applicator in which a housing is provided for a length of adhesive tape provided on a closed loop of backing material. The adhesive tape is drawn from the backing material and through an opening for application of the tape. The rear surface of the tape is contacted by a depressor formed as an extension to the housing, using which a user can apply pressure to the tape during dispensing. Another example of a tape dispenser is disclosed in US-A-4447482.

US-A-5685833 discloses an adhesive bandage provided in a strip of similar bandages and encapsulated in a protective covering. After application of a bandage, the protective covering is removable.

FR 2063743 shows a strip for diagnostic purposes.

According to a first aspect of the present invention, a tape stripping system comprises:
an ergonomically shaped handheld device (200) having internal motion generating means for imparting desired motion to a partially inserted component (213) at a distal tip thereof, and a distal tip surface having wall portions defining a connection aperture for connecting a rejuvenating surface component portion into the distal tip area aperture, and a handheld hand actuatable motion generating and control means; and
a distal component (213) having a first portion designed for insertion into a motion generating subsystem of the handheld device, and a second portion (217) having a shaped configuration to conform to a user's skin surface, with the said conformed surface (217) comprising means for placing a tape-like material on the said surface (217) to effect abrasion or polishing of the user's skin.

According to a second aspect of the present invention, a method of cosmetically rejuvenating skin comprising the steps of:
assessing the skin to be rejuvenated;
selecting a proper tape program for application and removal of tape to the skin; and
implementing the selected tape program.

Figure 1 is a perspective view of a handheld apparatus for self-administration of tape to skin.
Figure 2 is a section view of a tape roll taken along lines 2-2 of Figure 1.
Figure 3 is a plan view of an exemplary dispenser of tape rolls.
Figure 4 is diagram depicting a preferred system and methodology for administration of skin care using the tape removal concept of this invention.
Figure 5 is a bottom plan view of a tape dispenser.
Figure 6 is a perspective view of an embodiment of the invention, with a charging unit.
Figure 7 is a perspective view of an embodiment of the invention.

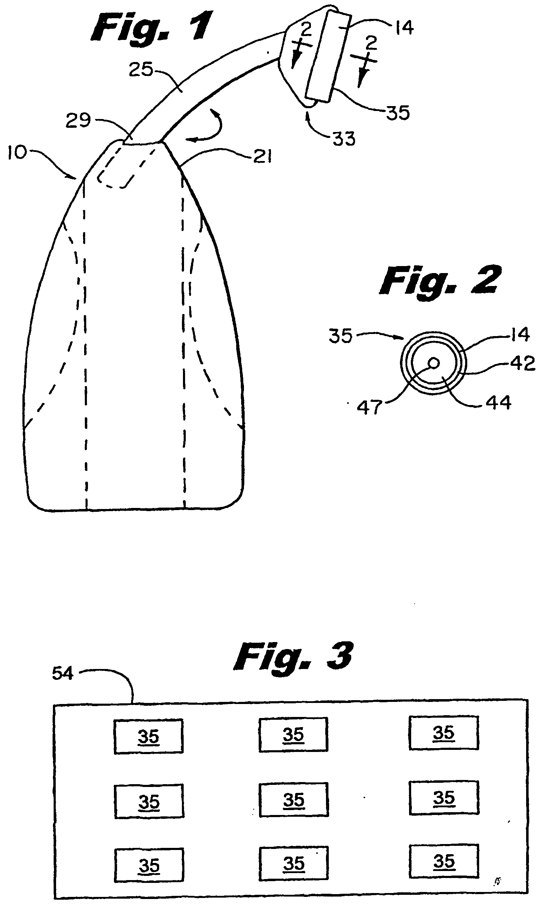

Figure 1 is a perspective view of a handheld apparatus 10 for self-administration of a tape-like product 14 to skin. Apparatus 10, also referred to as system 10, includes, in one embodiment, a handheld ergonomically structured device 21. Handheld device 21 comprises a body portion having a variety of optional shapes and configurations, as depicted by representative dashed lines, although not limited to said precise configurations. Most importantly, the body portion shape must conform to a comfortable gripping configuration of a user's hand when using the device for self-administration at or about the facial area, as well as other body portions. For this reason, it is recognized that extension 25 may be formed as a unitary part with the body portion, or it may be separably attached and/or inserted at various locations on body portion, such as at neck 29, or other locations. Suitable attaching and locking means are contemplated in the event that extension 25 forms a piece separate than the body portion. Regardless, a tape dispensing holder is positioned at a distal end 33 of extension 25. This feature comprises any of various retaining means suitable for holding and dispensing 2 rolled tape unit 35. It is appreciated that extension 25 may be made of a material that has a memory suitable for reconfiguring by each user, possibly through use of advanced technology material such as that similar to a nitinol-type of material or one with suitable reshaping characteristics, or it may be simply be manufactured to have a curvilinear or other shape suitable for a single design optimized for all users.

As shown in Figures 1 and 2, rolled tape unit 35 comprises a cylindrical-style roll of material forming at least one outer layer of tacky material, referred to herein as tape. The outer tacky material of rolled tape unit 35 is designed for dispensing along the surface of the user's skin, preferably facial skin, in order to remove detritus and other undesired material from the surface of the skin. Indeed, according to the various programs of usage and degree of tackiness of the tape, it is possible to remove both scales and external environmental pollutants as well as portions of the skin itself. In one embodiment it is also conceivable to have multiple layers of tape, for example, as shown in Figure 2 at layer 42. In this embodiment it is possible to utilize the same rolled tape unit 35, but extend its life by removing a used outer layer of tacky tape material 14 and exposing an unused tacky layer of tape material 42 for subsequent use, or at the subsequent use time. Layer or volume 44 of rolled tape unit 35 may simply comprise additional layers of tape or it may provide a spacer having various material properties. For example, one type of spacer may include a resilient material to reduce the rigidity and increase the tactile affection of the user and the product being applied to the user's skin.

Either a dimple or full channel may comprise the most efficacious means of allowing the rolled tape unit to function along with the distal end dispensing portion of the handheld device. Such dimple or channel is represented by element 47 in Figure 2.

Figure 3 discloses dispensing pack 54 designed for holding and dispensing one or more rolled tape units 35. In one embodiment, dispensing pack 54 may be structured with opening and closure layers similar to those packages known in the dispensing industry as blister packs. In this embodiment, the user simply presses on one side of dispensing pack 54 to release a single rolled tape unit 35 from the package. In that instance it is important that a cover layer (which is retaining the rolled tape unit within the package) to have a material or material coating suitable to prevent adherence to the rolled tape unit tacky layer. Alternatively, a non-tacky cover layer may be applied to each of the rolled tape units to facilitate storage, dispensing, and mounting on the handheld device prior to actual use against the skin. Then, in such an embodiment, the non-tacky layer is removed thus exposing the tacky tape material 14.

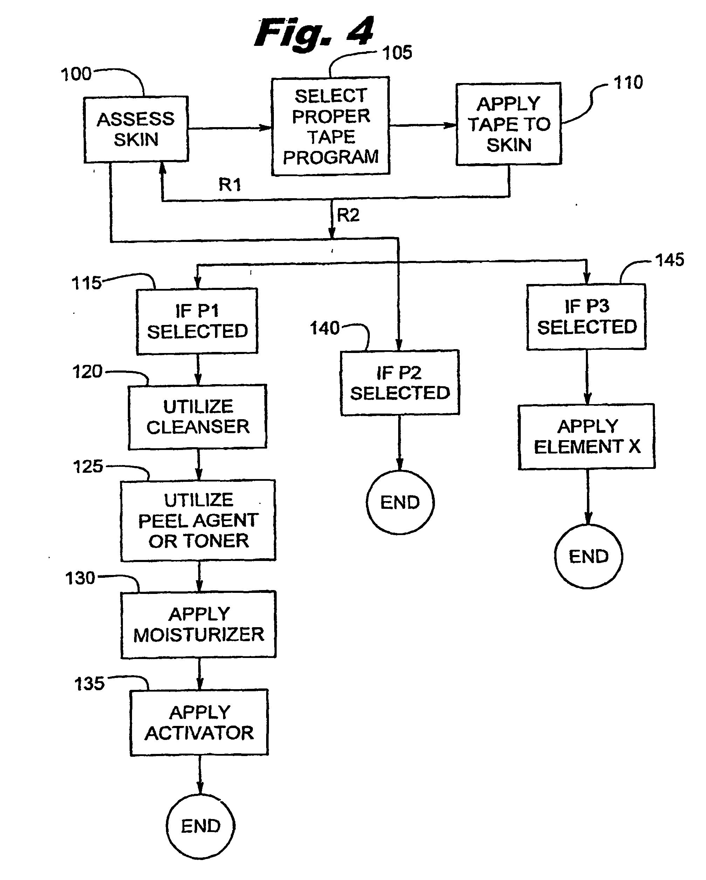

In the past, tape stripping technology has been limited to use by professionally trained medical and cosmetic practitioners. At least one objective of this invention is to facilitate use by the individual user independent of the inconvenience, high cost, and lack of privacy implications of requiring office visits and the like in order to receive the benefits of this technology. Rather, as shown in Figure 4, it is now possible to utilize the dispensing and individualized tape roller system disclosed in Figures 1-3 to allow individual users the chance to receive this treatment through self-administration. However, it is quite important to recognize that a methodology is recommended, and that such methodology has various options. As in most medical device, therapeutic, or other systems which relate to individual user preferences and morphology, there are various algorithms which apply to each individual user. Figure 4 demonstrates an example of the algorithmic approach to use of this technology.

Block 100 represents the proper first step or method which requires careful assessment of the user's skin history, skin type, current skin condition, and usage goals of the system. After such proper assessment is completed by the user, he or she then selects the proper tape program according to the assessment of block 100. For example, one type of program (referred to herein as "P1") comprises a comprehensive rejuvenation program as later discussed below. Alternatively, a second tape program (referred to herein as "P2") comprises use of a tape-like material 14 having certain medications, such as an antibacterial material or a cortisone material imbedded in the tape and configured for release upon application to the skin. Yet another tape program (referred to herein as "P3") which may be appropriate for the particular user includes application of one or more elements to the skin following the initial tape application and removal. Such elements are also discussed below.

Following assessment of the skin and selection of the proper tape program according to the goals of the user, then the user applies the tape-like material 14 to the skin at the appropriate areas using, in one embodiment, the handheld device and roller system shown in Figure 1. It is recognized that alternate dispensing means may be provided consistent with facilitating self-administration and ease of access to the various sites of interest on highly variable body morphologies. In a general sense however, this invention is designed to a great extent to facilitate the tape application to and removal from portions of a human facial skin area. Accordingly, a preferred ergonomic design may be found under the various options as shown in Figure 1 to facilitate gripping in a comfortable manner, particularly at an early or late part of a user's day when the hand may be less comfortable or more stiff for various reasons.

Following application of the tape to the skin, a skin assessment is again recommended under the pathway R1 shown in Figure 4. It is understood, however, that the skin assessment at this point in the procedure may be bypassed, particularly by experienced users. In this instance, pathway R2 directs the user to one of several program options P1, P2, or P3.

If the user selects tape program P1, then this program may include use of a cleanser 120, followed by a peel agent or toner 125, followed by an optional moisturizer 130, and then application of an activator 135. Examples of activators may include active lotions or materials such as those including vitamins or other rejuvenating elements. Alternatively, if the user selects tape program P2, then that user has chosen to utilize either a standard tape-like material 14 or a tape-like material 14 having additional medications imbedded or otherwise positioned on the tape. This latter configuration facilitates application of such medication while also removing the detritus or other tissue as discussed above during the same application of the tape. Regardless of which of the sub-options of step 140 of program 2 is selected, there is no need to proceed to any other substeps following application of the tape and removal of the tape from the skin.

However, if the user selects proper tape program P3 as shown at block 145, then the user may wish to followup the tape application and removal step with application of one of various types of elements, herein referred to as element X. For example, a topical anesthetic, a medication, a toning agent, a moisturer, an activator, or even simply a cleanser, or even a splash of water may be appropriate for use as element X. It is also recognized that element X may also include a second application and/or subsequent applications of the tape to the skin within the context of this disclosure.

It is recognized therefore that system 10 is useful for dispensing tape means having various adhesive or tacky features on the tape. This tape is designed to remove excess skin cells or other material from the surface of skin in order to rejuvenate and stimulate that skin. A disposable single use type application is possible within the scope of this invention, and is quite appropriate in view of the increased environmental challenges and air particulate encountered every day by users. This self-administration product is particularly useful for home users for smoothing, refreshing, and regenerating damaged skin at a much lower cost than lasers, chemical peels or micro-derm abrasion. Indeed, the advantages of the embodiments of this technology shown in this application are quite distinct even over specific tape stripping by professional medical or cosmetic providers in a medical or cosmetic office environment. Regardless, no system exists for teaching and guiding the self user in the efficacious uses of a tape stripping system. This invention overcomes the fallings of any prior art in providing simple and effective means for rejuvenating human skin in a manner appropriate to each individual user.

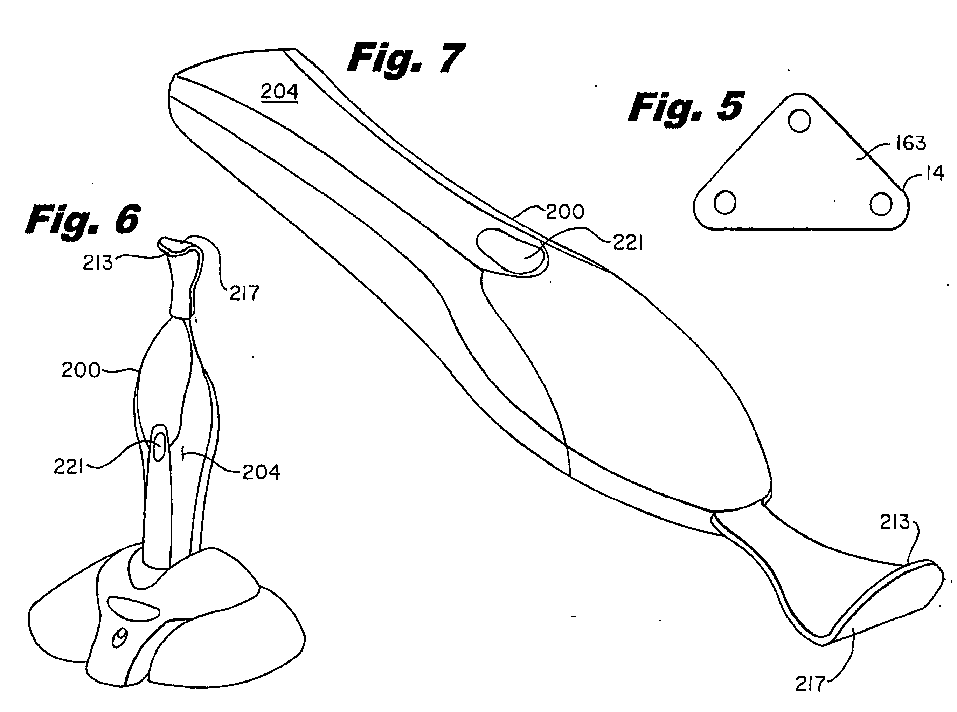

Further embodiments of handheld apparatus 10 may be configured in non-circular tape dispensing figurations, such as tape dispenser 163 shown in Figure 5, having a tape-like product 14. The devices of the invention disclosed herein may also function as a rolling tape application motion, or as circular, or reciprocating, provided that the required skin to tape interface is achieved.

Figures 6 and 7 illustrate an embodiment of the invention comprising handheld apparatus 200. Handheld apparatus 200 is designed to provide an ergonomic gripping handle 204, having internal charging and motion generating means, generally of conventional type similar to that found in mechanical or electrical toothbrush mechanisms, but with a capability of causing or imparting motion to an applicator tip or component 213. Applicator tip or component 213 may be configured so that it is removable and reconfigurable, as desired. In one embodiment, component 213 is designed to have a surface 217 configured to receive tape-like material, such as disclosed herein above in the tape stripping system, with the tape-like material suitable for abrading and applying medications as disclosed herein. In operation, device 200 is held by the user while component 217, which has previously been configured with a tape-like material having the appropriate abrasion characteristics, and the user activates the motion of the component 213 using various activation means such as, for example, activating mechanism 221. Following activation, component 213 is set in motion in either a reciprocating, circular or other fashion so as to effect the appropriate abrasion using the tape-like material on the user's skin. In an alternate use, the user may remove an abrading tape-like material and instead apply a buffing or polishing type strip on face 217 to effect further penetration or other desired effects upon the skin being treated. As shown in Figure 6, one embodiment of this device includes a charging stand which may be suitable for providing electric generating means to a rechargeable battery within the gripping portion of the device. Yet another embodiment includes, for example, a device 200 which does not have the internal mechanisms described herein above in relation to this handheld device but rather has a remote connection, such a wired connection, with a remotely configured motor or motion generating means which may be selectively applied by cord or wireless to the handheld device, thereby imparting the motion of applicator face 217 independent of the possible cumbersome size of an internally located motion generating mechanism.

It has also been appreciated by the inventors that the system, device components, and methods described above are useful for hair removal, drug delivery, and serum extraction for diagnostic and related purposes. Accordingly, the disclosure of the invention contemplates claims to these aspects of the invention as well.

## Claims

1. A tape stripping system for removal of excess material from the surface of a user's skin, comprising:
an ergonomically shaped handheld device (200) having internal motion generating means for imparting desired motion to a partially inserted component (213) at a distal tip thereof, and a distal tip surface having wall portions defining a connection aperture for connecting a rejuvenating surface component portion into the distal tip area aperture, and a handheld hand actuatable motion generating and control means; and
a distal component (213) having a first portion designed for insertion into a motion generating subsystem of the handheld device, and a second portion (217) having a shaped configuration to conform to a user's skin surface, with the said conformed surface (217) comprising means for placing a tape-like material on the said surface (217) to effect abrasion or polishing of the user's skin.

2. A tape stripping system according to Claim 1, in which the tape is adhesive or tacky.

3. A tape stripping system according to Claim 1 or Claim 2, in which the tape includes medications imbedded therein.

4. A tape stripping system according to Claim 3, in which the medication includes anti-bacterial or cortisone material.

5. A tape stripping system of any one of the preceding claims, in which the dispenser has holding means for selectively holding a tape unit.

6. The use of the tape stripping system according to any one Claims 1, 2 or 5 for the cosmetic cleaning and cosmetic rejuvenation of the skin, in which the tape does not include medications.

7. A method of cosmetically rejuvenating skin comprising the steps of:
assessing the skin to be rejuvenated;
selecting a proper tape program for application and removal of tape to the skin; and
implementing the selected tape program, in which the tape does not include medications.

8. The method of Claim 7, in which the selected tape program determines the motion of the device and the tape in contact with a user's skin.

## Patentansprüche

1. Streifenspendersystem zum Entfernen überschüssigen Materials von der Hautoberfläche eines Benutzers, umfassend:
ein ergonomisch geformtes handgehaltenes Gerät (200) mit einem internen, Bewegung generierendem Mittel zum Mitteilen gewünschter Bewegung an eine teilweise eingeschobene Komponente (213) an einem distalen Ende davon und eine distale Endoberfläche mit Wandteilen, die eine Anschlussöffnung zum Anschließen eines verjüngenden Oberflächenkomponententeils in die Öffnung des distalen Endbereichs und ein handgehaltenes, von Hand bedienbares, bewegungsgenerierendes Mittel und Kontrollmittel; und
eine distale Komponente (213) mit einem ersten Teil, der zum Einschieben in ein Bewegung generierendes Subsystem des handgehaltenen Geräts konzipiert ist und einem zweiten Teil (217) mit einer geformten Konfiguration, um sich der Hautoberfläche eines Benutzers anzupassen, wobei die besagte angepasste Oberfläche (217) Mittel zum Platzieren eines streifenartigen Materials auf die besagte Oberfläche (217) umfasst, um Abrieb oder Polieren der Haut des Benutzers zu bewirken.

2. Streifenspendersystem nach Anspruch 1, in dem der Streifen haftend oder klebrig ist.

3. Streifenspendersystem nach Anspruch Claim 1 oder Anspruch 2, in dem der Streifen darin eingebettete Medikamente einschließt.

4. Streifenspendersystem nach Anspruch 3, in dem das Medikament antibakterielles oder Cortisonmaterial einschließt.

5. Streifenspendersystem nach einem beliebigen der vorherigen Ansprüche, in dem der Spender Haltemittel zum selektiven Halten einer Streifeneinheit aufweist.

6. Gebrauch des Streifenspendersystems nach einem beliebigen der Ansprüche 1, 2 oder 5 für die kosmetische Reinigung und kosmetische Verjüngung der Haut, bei dem der Streifen keine Medikamente einschließt.

7. Methode zum kosmetischen Verjüngen von Haut, die folgende Schritte umfasst:
Beurteilen der zu verjüngenden Haut;
Auswählen eines geeigneten Streifenprogramms für das Aufbringen von Streifen auf die und deren Entfernen von der Haut; und
Umsetzen des ausgewählten Streifenprogramms, bei dem der Streifen keine Medikamente einschließt.

8. Methode des Anspruchs 7, bei der das ausgewählte Streifenprogramm die Bewegung des Geräts und des mit der Haut des Benutzers in Kontakt befindlichen Streifens bestimmt.

## Revendications

1. Un système d'arrachement de ruban pour retirer un excès de matière de la surface de la peau d'un utilisateur, comprenant :
un dispositif tenu à la main de forme ergonomique (200) ayant un mécanisme produisant un mouvement interne afin d'impartir le mouvement souhaité à un composant partiellement inséré (213) à une pointe distale de celui-ci et une surface de pointe distale ayant des parties parois définissant une ouverture de raccordement pour raccorder une partie composant de surface de rajeunissement dans l'ouverture de la zone de la pointe distale et un mécanisme tenu à la main, actionnable à la main, produisant et contrôlant un mouvement ; et
un composant distal (213) ayant une première partie conçue pour l'insérer dans un sous-système produisant un mouvement du dispositif tenu à la main et une deuxième partie (217) ayant une configuration formée pour se conformer la surface de la peau d'un utilisateur, avec ladite surface conformée (217) comprenant un mécanisme pour placer une matière de type ruban sur ladite surface (217) pour effectuer l'abrasion ou le polissage de la peau de l'utilisateur.

2. Un système d'arrachement de ruban selon la revendication 1, où le ruban est adhésif ou collant.

3. Un système d'arrachement de ruban selon la revendication 1 ou la revendication 2, où le ruban inclut des médicaments intégrés à l'intérieur.

4. Un système d'arrachement de ruban selon la revendication 3, où le ruban inclut une matière antibactérienne ou cortisonique.

5. Un système d'arrachement de ruban selon l'une quelconque des revendications précédentes, où le distributeur a un mécanisme de maintien pour maintenir une unité de ruban de manière sélective.

6. L'utilisation du système d'arrachement de ruban selon l'une quelconque des revendications 1, 2 ou 5 pour le nettoyage cosmétique et le rajeunissement cosmétique de la peau, où le ruban n'inclut pas de médicaments.

7. Un méthode pour rajeunir la peau cosmétiquement comprenant les étapes de :
l'évaluation de la peau à rajeunir ;
la sélection d'un programme de ruban approprié à l'application et au retrait du ruban de la peau ; et
la mise en oeuvre du programme de ruban sélectionné, où le ruban n'inclut pas de médicaments.

8. La méthode selon la revendication 7, où le programme de ruban sélectionné détermine le mouvement du dispositif et du ruban en contact avec la peau d'un utilisateur.
